# EUROPÄISCHE PATENTSCHRIFT

(11) **EP 0 536 192 B1**
(45) Veröffentlichungstag und Bekanntmachung des Hinweises auf die Patenterteilung: **31.08.1994**
(21) Anmeldenummer: 91911248.2
(22) Anmeldetag: 15.06.1991
(51) Int. Cl.: C12N 15/80, C12P 25/00, C12N 1/14

(54) **NEUE PROMOTORREGION**
NEW PROMOTER REGION
NOUVELLE REGION PROMOTRICE

(30) Priorität: 25.06.1990 DE 4020181
(43) Veröffentlichungstag der Anmeldung: 14.04.1993
(73) Patentinhaber: BASF Aktiengesellschaft, 67063 Ludwigshafen (DE)
(72) Erfinder: KURTH, Roland, D-6703 Limburgerhof (DE); PHILIPPSEN, Peter, D-6300 Giessen (DE); STEINER, Sabine, D-6300 Giessen (DE); WRIGHT, Martin, C., D-7950 Biberach/Riss (DE)
(86) Internationale Anmeldenummer: EP9101116
(87) Internationale Veröffentlichungsnummer: WO9200379

(56) Entgegenhaltungen:
- YEAST, vol. 6, 31 July 1990, CHICHESTER, U.K. PAGE 244; S. STEINER ET AL.: "THE GENE FOR THE TRANSLATION ELONGATION FACTOR EF-1ALPHA OF THE FILAMENTOU S YEAST ASHBYA GOSSYPII AND THE USE OF ITS PROMOTOR IN TRANSFORMATION EXPERIMENTS" see the whole document
- CHEMICAL ABSTRACTS, vol. 78, no. 15, 16 April 1973, Columbus, Ohio, US; abstract No. 96073K, T. SZCZESNIAK ET AL. "BYOSYNTHESIS OF RIBOFLAVINE" page 350; see abstract.

## Beschreibung

Die vorliegende Erfindung betrifft eine Promotorregion aus Ashbya gossypii (= A.gossypii), Pilze, die mit dieser Promotorregion genetisch verändert wurden, und deren Verwendung.

A.gossypii wird zur fermentativen Produktion von Vitamin B₂ eingesetzt. Es ist wünschenswert, die Anwendung der für A.gossypii vorhandenen Fermentationstechnologie durch die Produktion von Proteinprodukten unter Verwendung gentechnischer Methoden zu erweitern. Zu diesem Zweck wird ein Expressionssystem für A.gossypii benötigt. Für einige höhere Ascomyceten wie Aspergillus niger wurden solche Systeme bereits beschrieben (Rambosek und Leach, CRC Critical Reviews in Biotechnology 6 (1987), 357-393). Dagegen liegen mit dem Hemiascomyceten A.gossypii, der einziger Vertreter seiner Gattung ist, bisher keine Erfahrungen auf dem Gebiet der Gentechnologie vor.

Essentieller Bestandteil eines Systems zur Expression von Genen, die für ein gewünschtes Produkt kodieren, ist die sogenannte Promotorregion, die
1) aus einem funktionellen Promotor, der für die Transkription des Gens unerläßlich ist, und
2) aus der 5'nicht-kodierenden Region (zwischen Promotor und Translationsstart), die nach Transkription in der mRNA für die Translation nötig ist,
besteht.

Gegenstand der Erfindung ist die Promotorregion des A.gossypii-TEF-Gens, welches den Translationselongationsfaktor EF-1α (= TEF-1α) kodiert.

Dieses Gen wird sehr stark exprimiert und besitzt daher eine sehr effiziente Promotorregion.

Die erfindungsgemäß erhaltene Promotorregion besitzt die im Sequenzprotokoll Nr. 1 angegebene Nukleotidsequenz. Da man die funktionellen Bereiche einer neuen und sequenzierten Promotorregion, die die Fähigkeit zur Initiation der Transkription und Translation besitzen, am 3'-Ende gut und am 5'-Ende weniger gut eingrenzen kann, ist nicht auszuschließen, daß die natürliche Promotorregion des A.gossypii-Gens in der Länge leicht von der angegebenen Sequenz abweicht.

Ein weiterer Gegenstand der Erfindung ist die Terminatorregion des A. gossypii -TEF-Gens, welches den Translationselongationsfaktor EF-1α (= TEF-1α) kodiert.

Die Terminatorregion kann zur effizienten Transkriptionstermination verwendet werden.

Die erfindungsgemäß erhaltene Terminatorregion besitzt die im Sequenzprotokoll Nr. 2, Position 1513-2095, angegebene Nukleotidsequenz. Auch 3'-terminale Verkürzungen dieser Sequenz kommen als Transkriptionsterminator in Betracht.

Die Terminatorregion kann in Verbindung mit der TEF-Promotorregion oder mit anderen homologen oder heterologen Promotoren verwendet werden.

Gegenstand der Erfindung sind weiter Pilze, die die oben genannte Promotorregion oder Teile davon und/oder die oben genannte Terminatorregion oder Teile davon enthalten.

Die Promtorregion kann insbesondere in folgende Pilze insertiert werden: Ashbya gossypii, mit Ashbya eng verwandte Arten, wie insbesondere Eremothecium ashbyi und mit Ashbya nicht verwandte Gattungen wie insbesondere Aspergillus und Neurospora.

Die neue Promotorregion läßt sich herstellen
a) durch Klonierung des Gens für den Translationselongationsfaktor EF-1α (TEF-Gen) aus A.gossypii einschließlich angrenzender DNA-Sequenzen und anschließende Spaltung,
b) durch Fusion von A.gossypii-DNA-Fragmenten an ein offenes Leseraster eines in A.gossypii selektierbaren Promotor-losen Gens, Isolierung stark exprimierender Transformanden und nachfolgende Selektion des TEF-Promotors,
c) durch chemische Synthese nach bekannten Methoden.

Die neue Promotorregion eröffnet die Möglichkeit, zusammen mit geeigneten Vektorsystemen, in A.gossypii und anderen Pilzen homologe und heterologe Proteine zur Überexpression zu bringen. Dabei kann es sich beispielsweise um konstitutiv verstärkte Expression von Genen der Vitamin B₂-Biosynthese, sowie Genen, die für die Überproduktion von Vitamin B₂ verantwortlich sind, handeln oder um die Überexpression und Isolierung von Proteinen, die wirtschaftlich von Bedeutung sind. Weiterhin kann mit Hilfe der neuen Promotorregion das posttranskriptionale Modifizierungspotential (z.B. Glykosilierung) von A.gossypii, das unter Umständen von dem anderer Pilze verschieden ist, ausgenutzt werden. Da nicht alle heterologen Proteine in ausreichenden Mengen durch die bisher verwendeten Systeme wie Aspergillus oder Saccharomyces hergestellt werden können, ist die Entwicklung von Expressionssystemen mit der effizienten TEF-Promotorregion für neue Wirtsorganismen (hier zum Beispiel A.gossypii) von großer Bedeutung.

### Beispiele

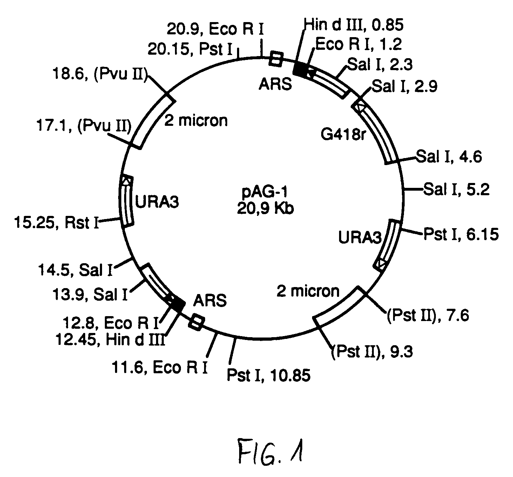

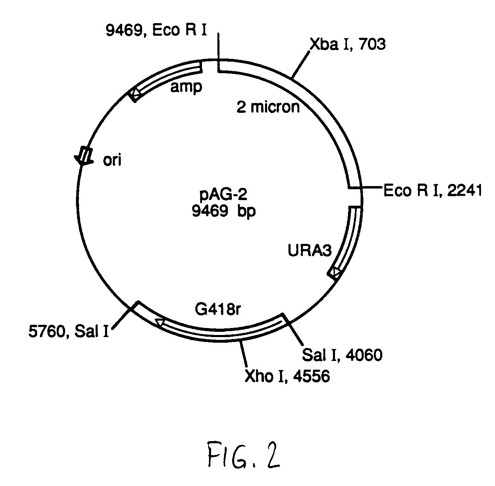

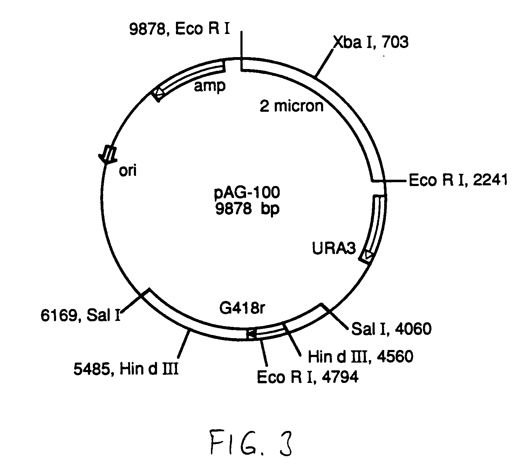

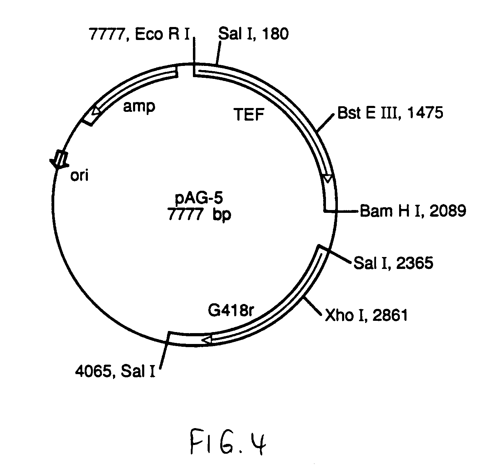

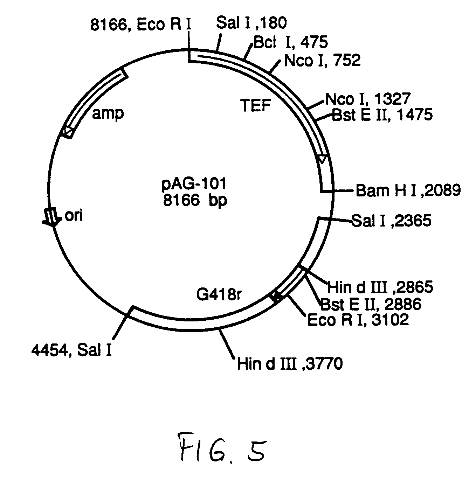

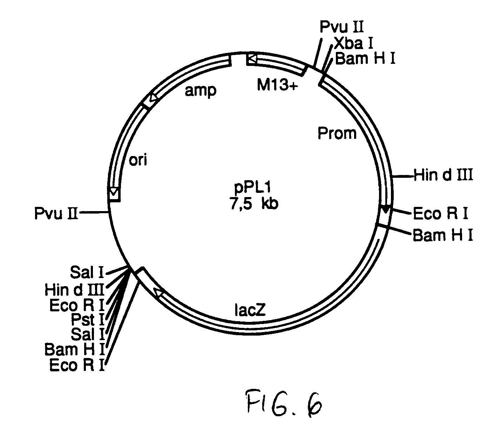

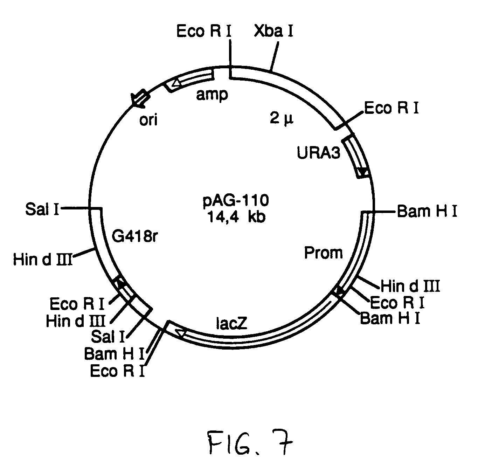

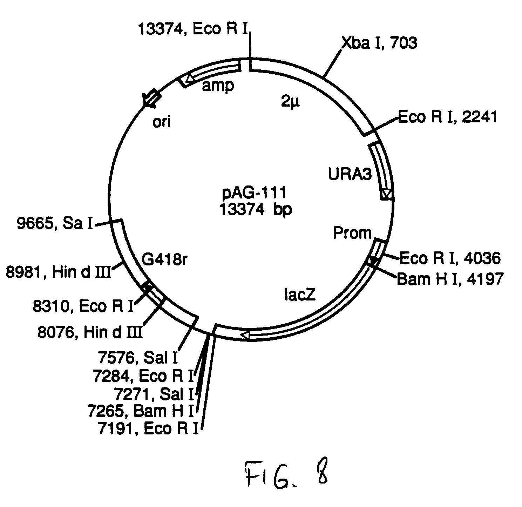

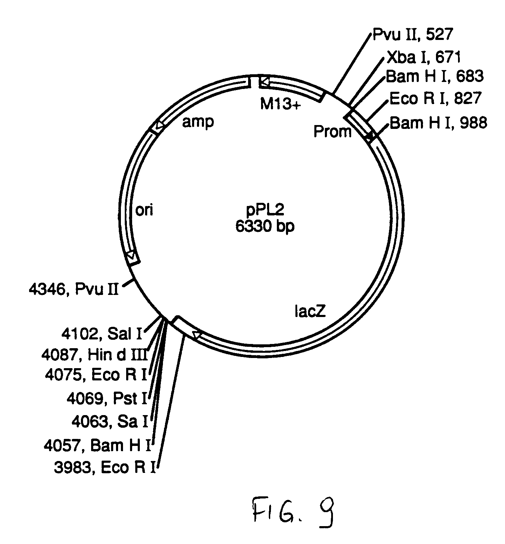

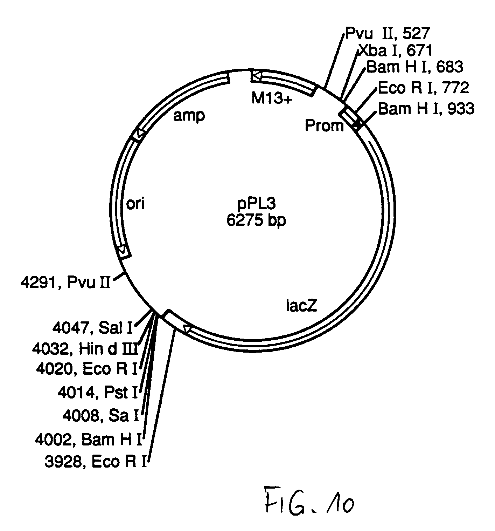

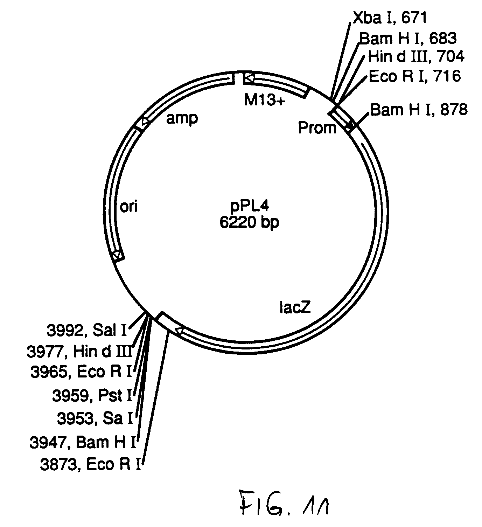

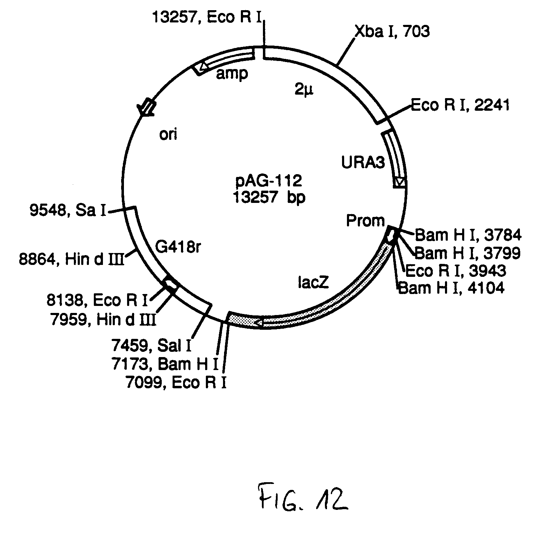

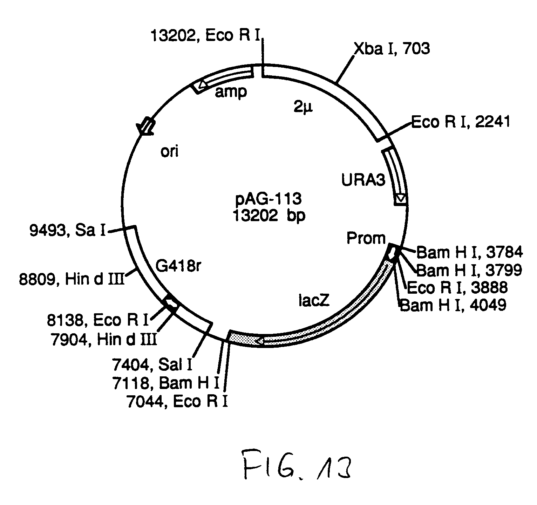

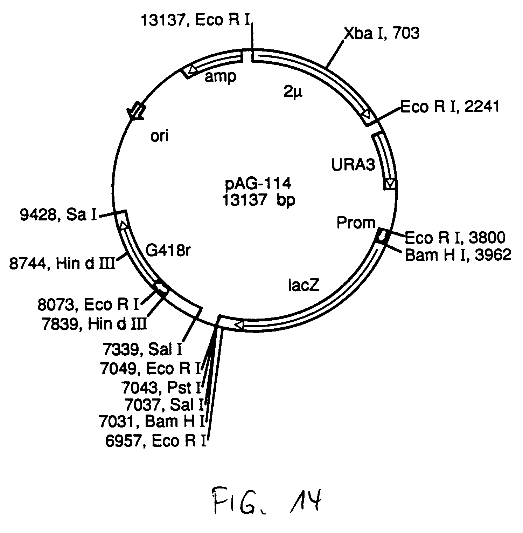

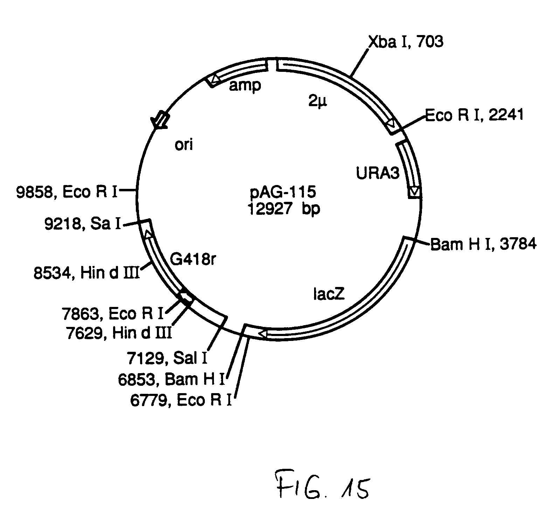

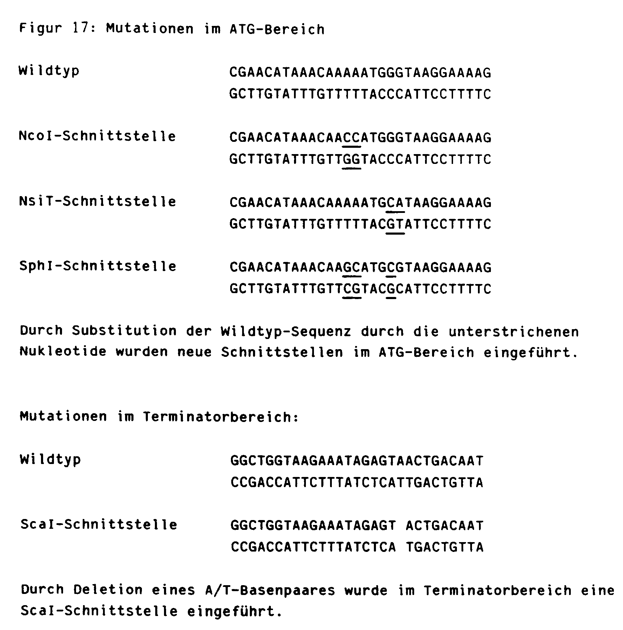

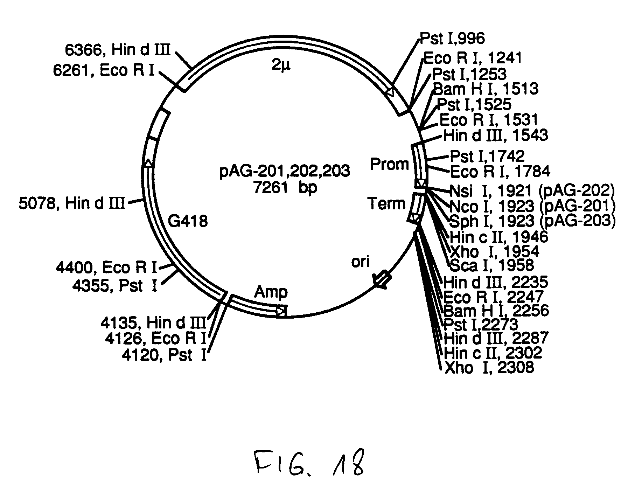

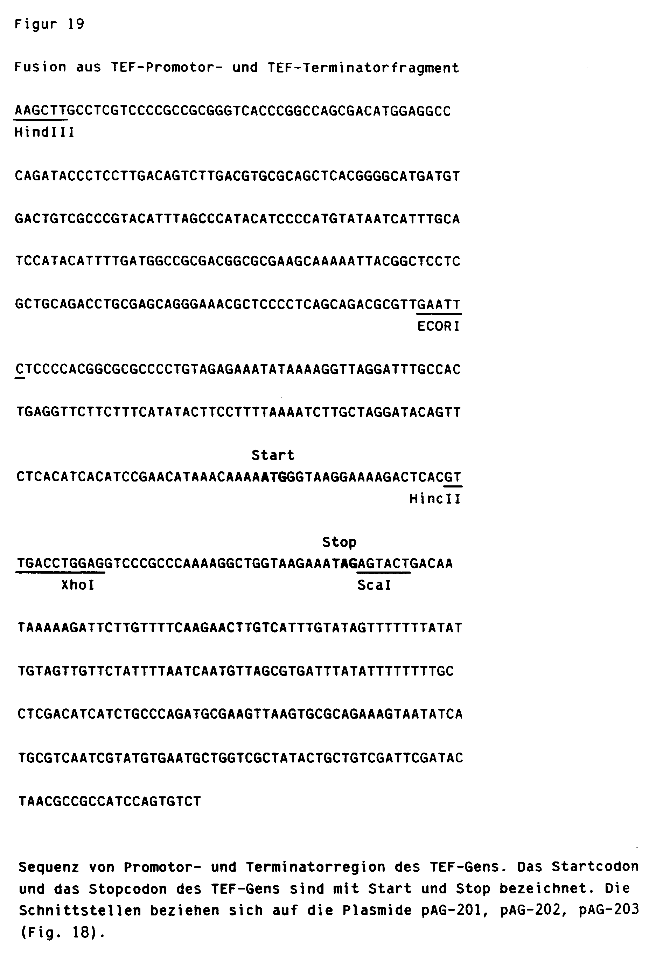

1. Isolierung des Ashbya gossypii-TEF-Gens
Aus A.gossypii-Mycel isolierte DNA wurde mit den Restriktionsendonukleasen EcoRI und BamHI geschnitten. DNA-Fragmente, die das TEF-Gen oder Teile davon tragen, wurden nach Größentrennung der Restriktionsfragmente in einer Agarose-Gelelektrophorese und eine nachfolgende Hybridisierung mit einer ³²P-markierten heterologen TEF-Genprobe identifiziert. Die TEF-Genprobe umfaßt die Nukleotide 363 bis 1235 des 1377 bp langen offenen Leserasters des S. cerevisiae TEF2-Gens (Schirmaier und Philippsen, EMBO J. 3 (1984), 3311-3315). Ein 4,6 kb langes EcoRI-Fragment und ein 6,4 kb langes BamHI-Fragment hybridisierten mit der heterologen TEF-Genprobe. Fragmente dieser Längenbereiche wurden aus Agarosegelen eluiert, in den mit EcoRI bzw. BamHI geschnittenen Vektor pUC8 (Vieira und Messing, Gene 19 (1982), 259-268) kloniert und in E.coli transformiert. Die Klone mit TEF-DNA wurden durch Koloniehybridisierung (Grunstein und Hogness, Proc. Natl. Acad. Sci. USA 72 (1975), 3961-3965) mit Hilfe der ³²P-markierten heterologen Probe erkannt. Die positiven Klone enthielten entweder das 4,6 kb lange EcoRI-Fragment oder das 6,4 kb lange BamHI-Fragment. Beide Klone überlappen in einem Bereich von 2,1 kb, der die Homologie zur TEF-Genprobe trägt und der sequenziert wurde (Sequenz Nr. 2). Dieses 2,1 kb lange Fragment enthält das offene Leseraster von 1377 bp, 136 bp der 5'-nichtkodierenden Region und 582 bp der 3'-nichtkodierenden Region. Über die EcoRI-Schnittstelle hinaus wurden weitere 278 bp der 5'-nichtkodierenden Region bis zu einer HindIII-Schnittstelle bestimmt. Anschließend wurde die Promotorregion als 403 bp langes HindIII/HincII-Fragment, das neben den 379 bp vor dem Startcodon noch die ersten 24 bp des offenen Leserasters des TEF-Gens trägt, isoliert und für die Konstruktionen von pAG-100 und pAG-101 eingesetzt (Sequenz Nr. 1).
2. Plasmidkonstruktionen
a) Der Vektor pAG-1 (Fig. 1) (hinterlegt DSM 6010), ein Derivat des Vektors pEX4 wurde gemäß Ernst und Chan, J.Bacteriol. 163 (1985), 8-14 hergestellt. pAG-1 enthält ein 1,7 kb SalI-Fragment mit dem für die Aminoglykosidphosphotransferase (APH(3')I) kodierenden Kanamycinresistenzgen des Transposons Tn903. Im ursprünglichen pEX4-Konstrukt wurde zunächst das 1695 bp PvuII-Fragment von Tn903 (Oka et al., J.Mol.Biol. 147 (1981), 217-226) in ein Plasmid mit aufgefüllten SalI-Schnittstellen einligiert. Die SalI-Schnittstellen bleiben dabei erhalten und das Resistenzgen kann als 1,7 kb SalI Fragment isoliert werden. pAG-1 enthält die Saccharomyces cerevisiae ARS-Elemente ARS1 und 2µ ARS und repliziert autonom in Ashbya gossypii.
b) pAG-2 (Fig. 2). Das 1,7 kb SalI-Fragment mit den Kanamycinresistenzgen wurde aus pAG-1 ausgeschnitten und in die SalI Schnittstelle des S. cerevisiae E. coli Shuttlevektors XEp24 (Botstein et al., Gene 8 (1979), 17-24; New England Biolabs Inc., Beverly, MA, USA, 1988-1989 Catalog, 112-113) eingefügt. Die Struktur des neu entstandenen Plasmides - pAG-2 - wurde durch Restriktionsendonukleasekartierung überprüft, wobei die im 1,7 kb SalI-Fragment gelegene XhoI-Schnittstelle zur Überprüfung der Insertorientierung benutzt wurde. pAG-2 enthält das Saccharomyces cerevisiae ARS-Element 2µ ARS und repliziert autonom in Ashbya gossypii.
c) pAG-100 (Fig. 3). In die XhoI Schnittstelle von pAG-2, die 30 bp in 3'-Richtung hinter dem Translationsstart des Kanamycinresistenzgens liegt, wurde nach Auffüllen der überstehenden Enden ein 403 bp langes HindIII/HincII-Fragment, das die Promotorregion und die ersten 24 bp des offenen Leserasters des Gens für den Translationselongationsfaktor EF-1α (TEF-Gen) aus A.gossypii enthält, eingefügt. Die Orientierung des Fragmentes in dem so entstandenen Plasmid pAG-100 wurde durch Restriktionsendonukleasekartierung mit HindIII überprüft. Durch Einfügen des 403 bp langen Fragmentes wurden die 10 N-terminalen Aminosäuren der APH(3')I durch die ersten 8 Aminosäuren des A.gossypii Translationselongationsfaktors EF-1α ersetzt. Entfernen oder Austausch der ersten 19 Aminosäuren der APH(3')I durch andere Aminosäuren führt nicht zu einem Verlust der Aktivität (Chen und Fukuhara, Gene 69 (1988), 181-192). Die Sequenz des SalI-Fragmentes nach Einfügen der TEF-Promotorregion zeigt Sequenz Nr. 2. pAG-100 enthält das Saccharomyces cerevisiae ARS-Element 2µ ARS und repliziert autonom in Ashbya gossypii.
d) pAG-5 (Fig. 4). Das 1,7 kb Fragment mit dem Kanamycinresistenzgen aus pAG-1 wurde in die SalI-Schnittstelle von pBR322 (Bolivar et al., Gene 2 (1977), 95-113) subkloniert. Das entstandene Plasmid - pJL3A - enthält im pBR322-Anteil jeweils eine BamHI- und eine EcoRI-Schnittstelle, so daß pJL3A durch Doppelverdau in ein 375 bp und ein 5688 bp Fragment zerlegt wird. Das große Fragment wurde mit einem 2,1 kb EcoRI/BamHI A.gossypii Fragment, das das offene Leseraster des Gens für den Translationselongationsfaktor EF-1α (TEF-Gen) enthält (Sequenz Nr. 1), ligiert. Das entstandene Plasmid wurde als pAG-5 bezeichnet. pAG-5 enthält keine Saccharomyces cerevisiae ARS-Elemente.
e) pAG-101 (Fig. 5). In die im offenen Leseraster des Kanamycinresistenzgens gelegene XhoI Schnittstelle wurde - wie für die Konstruktion von pAG-100 beschrieben - das 403 bp HindIII/HincII-Fragment mit der Promotorregion und den ersten 24 bp des offenen Leserasters des TEF-Gens aus A.gossypii eingefügt. Das so entstandene Plasmid erhielt die Bezeichnung pAG-101. pAG-101 enthält keine Saccharomyces cerevisiae ARS-Elemente.
f) pBKS1871 (Vorläuferplasmid für die TEF-Promotor-1acZ-Fusion): In die PstI Schnittstelle des Plasmides pBKS⁺ (Short et al., Nucleic Acid Res., 16 (1988), 7583-7600) wurde ein 3113 bp langes PstI Fragment aus dem Plasmid pMC1871 (Shapira et al., Gene, 25 (1983), 71-82) kloniert. Das Fragment trägt das offene Leseraster des 1acZ-Gens aus E.coli (Kalnins et al., EMBO J., 2 (1983), 593-597), dem die ersten sieben Codons fehlen.
g) pPL1 (Fig. 6). pBKS1871 wurde an der SmaI Schnittstelle vor dem 1acZ-Gen linearisiert. In das linearisierte Plasmid wurde ein 1500 bp HincII-Fragment einkloniert, das den TEF-Promotor und angrenzende Sequenzen einschließlich der ersten acht Codons des TEF-Gens aus A.gossypii trägt. Dadurch entstand ein offenes Leseraster, das für eine β-Galactosidase codiert, deren erste sieben Aminosäuren durch die ersten acht Aminosäuren des EF-1α aus A.gossypii ersetzt sind. Aus diesem Plasmid konnten TEF-Promotor-1acZ-Fusionen mit verschieden langen Bereichen des TEF-Promotors isoliert werden.
h) pPL2 (Fig. 9). pBKS1871 wurde an der SmaI-Schnittstelle vor dem 1acZ-Gen linearisiert. In das linearisierte Plasmid wurde ein 294 bp langes RsaI/HincII-Fragment einkloniert, das Teile des TEF-Promotors (270 bp) sowie die ersten acht Codons des TEF-Gens aus A. gossypii enthält (24 bp).
i) pPL3 (Fig. 10). pBKS1871 wurde an der SmaI-Schnittstelle vor dem 1acZ-Gen linearisiert. In das linearisierte Plasmid wurde ein 239 bp langes HaeIII/HincII-Fragment einkloniert, das die ersten acht Codons des TEF-Gens (24 bp) und 215 bp der in 5'-Richtung vor dem Startcodon gelegene Bereiche der nichttranslatierten Region enthält.
j) pPL4 (Fig. 11). pBKS1871 wurde an der SmaI-Schnittstelle vor dem 1acZ-Gen linearisiert. In das linearisierte Plasmid wurde ein 158 bp langes EcoRI/HincII-Fragment einkloniert, das die ersten acht Codons des TEF-Gens und 134 bp der in 5'-Richtung vor dem Startcodon gelegenen Bereiche der nichttranslatierten Region enthält.
k) pAG-110 (Fig. 7). Durch Spaltung von pPL1 mit XbaI und SalI wurde ein 4600 bp Fragment, das die Fusion des 1500 bp langen TEF-Promotorfragmentes mit dem lacZ Gen trägt, isoliert. Dieses Fragment wurde nach Auffüllen der überstehenden Enden in die aufgefüllte BamHI Schnittstelle von pAG-100 einkloniert.
l) pAG-111 (Fig. 8). Durch Spaltung von pPL1 mit HindIII wurde ein 3509 bp langes Fragment isoliert. In diesem Fragment ist die TEF-Promotorregion um 1100 bp verkürzt. Sie entspricht damit der Promotorregion, die in pAG-100, pAG-101, pAG-110 und pAG-111 die Transkription des G418 Resistenzgens kontrolliert. Nach Auffüllen der überstehenden Enden wurde das 3509 bp lange Fragment in die aufgefüllte BamHI Schnittstelle von pAG-100 einkloniert.
m) pAG-112 (Fig. 12). Nach Spaltung von pPL2 mit XbaI und SalI wurde ein 3392 bp langes Fragment, das die Fusion des 294 bp langen Promotorfragmentes mit dem 1acZ-Gen trägt, isoliert und nach Auffüllen der überstehenden Enden in die aufgefüllte BamHI-Schnittstelle des Plasmids pAG-100 eingesetzt.
n) pAG-113 (Fig. 13). Nach Spaltung von pPL3 mit XbaI und SalI wurde ein 3337 bp langes Fragment, das die Fusion des 239 bp langen Promotorfragmentes mit dem 1acZ-Gen trägt, isoliert und nach Auffüllen der überstehenden Enden in die aufgefüllte BamHI-Schnittstelle des Plasmids pAG-100 eingesetzt.
o) pAG-114 (Fig. 14). Nach Spaltung von pPL4 mit HindIII wurde ein 3273 bp langes Fragment, das die Fusion des 158 bp langen Promotorfragmentes mit dem 1acZ-Gen trägt, isoliert und nach Auffüllen der überstehenden Enden in die aufgefüllte BamHI-Schnittstelle des Plasmids pAG-100 eingesetzt.
p) pAG-115 (Fig. 15). Nach Spaltung von pBKS 1871 mit BamHI wurde ein 3069 bp langes Fragment isoliert, das das offene Leseraster des 1acZ-Gens trägt, wobei die ersten sieben Codons des offenen Leserasters fehlen und kein Promotorfragment vor das offene Leseraster fusioniert wurde. Dieses Fragment wurde in die BamHI-Schnittstelle des Plasmides pAG-100 eingefügt.
q) pAG-120.pBIIKS⁻ (Short et al., Nucleic Acid Res. 16 (1988), 7583-7600) wurde mit SspI und ScaI gespalten und ein 2084 bp langes Fragment isoliert. YEP24 (Botstein et al., Gene 8 (1979), 17-24) wurde mit Scal und ClaI gespalten und ein 2782 bp großes Fragment isoliert. Dieses wurde nach Auffüllen der überstehenden Enden mit dem 2084 bp langen ScaI/SspI-Fragment aus pBIIKS⁻ ligiert, so daß wieder ein vollständiges Ampicillinresistenzgen entstand (ScaI schneidet in pBIIKS⁻ und YEP24 im Ampicillinresistenzgen).
r) pAG-121.pAG-100 wurde mit SalI und HindIII geschnitten und ein 669 bp langes Fragment, das einen Teil des G418-Resistenzgens trägt, isoliert. Dieses wurde in das SAlI/HindIII geschnittene Plasmid pBIISK⁺ (Short et al., Nucleic Acid Res. 16 (1988), 7583-7600) einkloniert.
s) pAG-122.pAG-100 wurde mit HindIII geschnitten und ein 940 bp langes Fragment, das einen Teil des G418-Resistenzgens unter Kontrolle des TEF-Promotors trägt. Dieses wurde in das mit HindIII geschnittene Plasmid pAG-121 so eingefügt, daß ein vollständiges G418-Resistenzgen entstand. Transformation dieses Plasmides in E. coli erlaubt eine Transformandenselektion auf Kanamycin-haltigem Medium.
t) pAG-123.pAG-122 wurde mit SalI und BamHI geschnitten und ein 1639 bp langes Fragment isoliert, das das G418-Resistenzgen unter Kontrolle des TEF-Promotors trägt. Dieses wurde in das mit ScaI geschnittene Plasmid pAG-120 eingefügt, wodurch eine Selektion von E. coli Transformanden auf Kanamycin-haltigem Medium ermöglicht wurde.
u) pAG-130.pBIIKS⁺ (Short et al., Nucleic Acid Res. 16 (1988), 7583-7600) wurde mit HindIII und HincII gespalten und das 403 bp lange HindIII/HincII-TEF-Promotorfragment eingefügt.
v) pAG-131. Aus dem Clon, der das 2,1 kb große Fragment genomischer A. gossypii DNA trägt, auf dem das TEF-Gen enthalten ist, wurde ein 260 bp großes HaeIII/AccI-Fragment isoliert, das 25 Nukleotide des 3'-Endes des TEF-Gens sowie in 3'-Richtung daran anschließende Bereiche enthält (Terminatorfragment). Dieses Fragment wurde nach Auffüllen der überstehenden Enden in das mit HincII gespaltene Plasmid pBIIKS⁻ (Short et al., Nucleic Acid Res. 16 (1988), 7583-7600) eingefügt.
w) pAG-132. pag-130 wurde mit ScaI und XhoI geschnitten und ein 2248 bp großes Fragment isoliert. pAG-131 wurde ebenfalls mit ScaI und XhoI gespalten und ein 1442 bp großes Fragment isoliert, das so mit dem 2248 bp Fragment aus pAG-103 ligiert wurde, daß erneut ein vollständiges Ampicillinresistenzgen entstand.
x) M13PT. pAG-132 wurde mit BamHI gespalten und ein 752 bp großes Fragment isoliert, das die Fusion aus TEF-Promotor- und TEF-Terminatorfragment enthält. Dieses wurde in die BamHI-Schnittstelle von M13mp9 einkloniert.
y) M13PT1, M13PT2, M13PT3.
   M13PT wurde durch Oligonukleotid-gerichtete Mutagenese (Kramer et al., Nucl.Acid Res. 24 (1984), 9441-9556) so verändert, daß hinter dem Stopcodon des TEF-Gens (im Terminatorfragment) eine ScaI-Schnittstelle und im Startcodon des TEF-Gens (im Promotorfragment) eine NcoI-Schnittstelle (M13PT1), eine NsiI-Schnittstelle (M13PT2) oder eine SphI-Schnittstelle (M13PT3) hergestellt wurde (Fig. 17).
z) pAG-201. pAG-202 pAG-203 (Fig. 18)
   M13PT1, M13PT2 und M13PT3 wurden mit BamHI gespalten und aus der Spaltung wurde das 751 bp große Fragment mit Promotor- und Terminatorregion des TEF-Gens isoliert. Dieses TEF-Signalsequenz wurde in die BamHI-Schnittstelle des Plasmides pAG-123 eingefügt und ergab das Plasmid pAG-201. Nach dem gleichen Verfahren wurden aus M13PT2 das Plasmid pAG-202 und aus M13PT3 das Plasmid pAG-203 konstruiert.

3. Transformation von A.gossypii mit TEF-Promotorregion-Plasmiden
Die Transformationen erfolgten nach folgendem Schema:
- 200 ml MA2 mit ca. 1-2x10⁷ Sporen beimpfen
- 32-40 h bei 27°C mit 350 Upm in Schikanenkolben inkubieren
- Myzel mit Saugfiltration abfiltrieren und 1x in 30 ml H₂O waschen
- Frischgewicht bestimmen (ca. 2-3 g)
- Myzel in 30 ml SD suspendieren und 30 min bei 30°C in Schüttler inkubieren
- Myzel in 5-10 ml SPEZ pro g Frischgewicht suspendieren
- Bei 30°C in Wasserbadschüttler inkubieren, Protoplastierung mikroskopisch überprüfen (nach 30 min sollte ein Protoplastierungsgrad von über 90 % erreicht sein)
- Protoplastensuspension über Glasfilter (Schott, Porosität 1) filtrieren)
- Filtrat 5 min zentrifugieren (Sorvall SM24 Rotor, 1800 Upm)
- Sediment 1x in 20 ml ST und 1x in 20 ml STC waschen
- Protoplasten in 20 ml STC suspendieren und Titer in Zählkammer bestimmen
- Nach Zentrifugation Protoplasten zu einer Dichte von 4x10⁸/ml in STC resuspendieren
- 100 µl Protoplastensuspension zu DNA in maximal 15 µl TE geben und mischen (DNA-Mengen: für replizierende TEF-Promotorregion-Plasmide: 1-10 µg; für integrative Transformation mit linearisierten TEF-Promotorregion-Plasmiden: 15-20 µg)
- 15 min bei Raumtemperatur inkubieren
- Vorsichtig 1 ml PTC40 zugeben und durch Invertieren mischen
- 5 min zentrifugieren (Heraeus Biofuge A, 1500 Upm)
- überstand vorsichtig abziehen und Sediment in 1 ml SMTCI suspendieren
- 3 h bei 27°C inkubieren, ca. alle 45 min durch Invertieren mischen
- Nach Zentrifugation Sedimente in 1 ml SM suspendieren
- Suspension mit 9 ml SMA2 Toplayer mischen und auf SMA2 Platte geben (20 ml SMA2-Agar pro Platte)
- Platten 18 h bei 27°C inkubieren
- Platten mit G418 überschichten (0,54 ml G418 Stammlösung +0,46 ml H₂O + 6 ml 0,5 % Agarose (in H₂O, vorgewärmt auf 42°C))
- Platten bei 27°C weiter inkubieren, Transformanden sind bei replizierenden Plasmiden nach 2-3 Tagen, bei Integration nach 3-6 Tagen sichtbar
Medien und Lösungen

| | | | |
|---|---|---|---|
| Medien: | MA2: | Pepton (Gibco Caseinhydrolysat, No. 140): | 10 g/l |
| | | Hefeextrakt (Gibco) : | 1 g/l |
| | | Glucose | 10 g/l |
| | | myo-Inositol | 0,3 g/l |
| | SMA2-Agar: | Sorbitol : | 1 M |
| | | Pepton : | 10 g/l |
| | | Hefeextrakt : | 1 g/l |
| | | Glucose | 20 g/l |
| | | myo-Inositol | 0,3 g/l |
| | | Agar (Gibco) | 12 g/l |
| SMA2-Toplayer: Wie SMA2-Agar, statt Agar 0,8 % Agarose | | | |

Lösungen:
- SD:: 1M Sorbitol; 50 mM Dithiothreitol
- SPEZ:: 1M Sorbitol; 10 mM Na-Phosphatpuffer pH 5,8; 10 mM EDTA; 2 mg/ml Zymolyase 20 T (Seikagaku Kogyo Co., Tokyo)
- ST:: 1M Sorbitol; 10 mM Tris-Cl pH 8
- STC:: 1M Sorbitol; 10 mM Tris-Cl pH 8; 10 mM CaCl₂
- TE:: 10 mM Tris-Cl; 1 mM EDTA
- PTC40:: 40 % (w/v) Polyethylenglykol 4000 (Merck); 10 mM Tris-Cl pH 8; 10 mM CaCl₂
- SMTCI:: 50 % SM (siehe unten); 50 % STC; 0,03 g/l myo-Inositol
- SM:: 50 % 2M Sorbitol; 50 % MA2
- G418-Stammlösung:: 20 mg/ml G418 (Geneticin, Gibco) in H₂O

4. Transformationsergebnisse mit TEF-Promotorregionplasmiden
Die Ergebnisse von verschiedenen Transformationen, die nach Beispiel 3 durchgeführt wurden, sind in Tabelle 1 zusammengefaßt. Bei allen Experimenten wurden Transformanden mit einer G418-Konzentration von 0,3 mg/ml pro Transformationsplatte selektiert. Bei dieser G418-Konzentration wird das Wachstum von A.gossypii Myzel vollständig inhibiert. Bei Transformation mit den rekombinanten DNA-Vektoren pAG-1 und pAG-2, bei denen das G418-Resistenzgen unter Kontrolle des ursprünglichen bakteriellen Promotors und nicht unter Kontrolle der TEF Promotorregion steht, entstanden bei dieser Konzentration keine Transformanden. Um mit diesen rekombinanten DNA Vektoren Transformanden zu erhalten, darf die G418-Konzentration 0,1 mg/ml pro Transformationsplatte nicht überschreiten. Bei dieser Konzentration sind bis zu 80 % der auftretenden Kolonien keine Transformanden.

**Tabelle 1**

| Transformationsergebnisse | | | | |
|---|---|---|---|---|
| Experiment | Plasmid | DNA pro Transf., µg | Transformanden pro µg DNA | Transformanden pro lebensfäh. Protoplasten |
| 1 | pAG-1 | 10 | 0 | 0 |
| 1 | pAG-2 | 10 | 0 | 0 |
| 1 | pAG-100 | 10 | 10 | 1,2 x 10⁻⁴ |
| 2 | pAG-100 | 0,1 | 10 | 1,6 x 10⁻⁵ |
| 3 | pAG-100 | 1 | 3 | 3,4 x 10⁻⁴ |
| 3 | pAG-101, mit BamHI linearisiert | 20 | 0,05 | 1,1 x 10⁻⁵ |

5. Transformationsergebnisse mit lacZ-Plasmiden
Um die Funktionsfähigkeit des TEF-Promotors weiter zu untersuchen, wurden Derivate des Plasmides pAG-100 konstruiert, in denen das Gen für die β-Galactosidase aus E.coli (lacZ-Gen) unter Kontrolle des TEF-Promotors steht. Hierfür wurden verschiedene Bereiche der Promotorregion des TEF-Gens vor das offene Leseraster des lacZ-Gens fusioniert, wobei die ersten sieben Codons des lacZ-Gens durch die ersten acht Codons des TEF-Gens ersetzt wurden. Das Plasmid pAG-110 trägt ein ca. 1,5 kb langes HincII TEF-Promotorfragment vor dem lacZ-Gen und das Plasmid pAG-111 das 403 bp lange HindIII/HincII-TEF-Promotorfragment, das bereits für die Konstruktionen von pAG-100 und pAG-101 eingesetzt wurde. Das Plasmid pAG-112 trägt ein 294 bp langes TEF-Promotorfragment, Plasmid pAG-113 ein 239 bp langes TEF-Promotorfragment und pAG-114 ein 158 bp langes TEF-Promotorfragment.
Zusätzlich wurde als Kontrollplasmid pAG-115 konstruiert, das das offene Leseraster des lacZ-Gens ohne Fusion an ein Promotorfragment trägt.
Nach Transformation dieser Plasmide in A.gossypii wurde die Expression des lacZ-Gens mittels eines Farbtests überprüft. Die vom lacZ-Gen codierte β-Galactosidase spaltet X-Gal (5-Brom-4-chlor-3-indoyl-β-D-galactosid) in den blauen Farbstoff 5-Brom-4-chlor-indigo. pAG-110-, pAG-111- und pAG-112-Transformanden bildeten auf Medium, das X-Gal (Miller, Experiments in Molecular Genetics, Cold Spring Harbor, New York 1972, 48) in einer Konzentration von 100µg/ml enthielt, blaue Kolonien. Bei Transformanden, die pAG-113, pAG-114 oder pAG-115 enthielten, war keine Blaufärbung zu sehen.

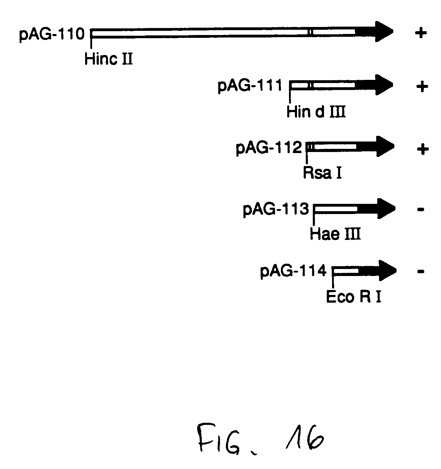

Einen Überblick über die verschiedenen TEF-Promotorfragmente, die vor das lacZ-Gen fusioniert wurden, zeigt Fig. 16. Ein + steht für eine Blaufärbung der Kolonien auf X-Gal-haltigem Medium, ein - für keine sichtbare Blaufärbung.

Für eine weitere Untersuchung der β-Galactosidase-Expression wurde die β-Galactosidaseaktivität aus Flüssigkulturen von pAG-110-, pAG-111-, pAG-112-, pAG-113-, pAG-114- und pAG-115-Transformanden bestimmt. Hierfür wurde das Mycel mit Glaskugeln aufgebrochen (Rose, M.; Casadaban, M.J. and Botstein, D., Proc. Natl. Acad. Sci. USA Vol. 78, No. 4 (1981), 2460-2464). 0,5 g Mycel, das in MA2-Flüssigmedium mit 200 µg/ml G 418 gewachsen war, wurden in 0,1 mM Tris, pH 8,0/20 % (vol/vol) Glycerin/1 mM DTT/1 mM PMSF aufgenommen und nach Zugabe von 0,5 g Glaskugeln (Durchmesser 0,45-0,5 mm) bei -20°C weggefroren. Für den Aufschluß des Mycels wurde 12 mal 15 sec bei 4°C heftig geschüttelt (Vortex). Anschließend wurde zweimal für 20 min bei 10000 rpm zentrifugiert (Sorvall Kühlzentrifuge). Die Überstände wurden 1:10 bzw. 1:20 in Z-Puffer (0,06 M Na₂HPO₄/0,04 M NaH₂PO₄/0,01 M KCl/0,001 M MgSO₄/0,05 M β-Mercaptoethanol) verdünnt. In den verdünnten Protein-Rohextrakten wurde die β-Galactosidaseaktivität durch Spaltung von o-Nitrophenyl-β-D-galactopyranosid bestimmt (Miller, Experiments in Molecular Genetics, Cold Spring Harbor, New York 1972, 353ff). Die Enzymaktivität wurde in Relation zu der Proteinkonzentration im Rohextrakt gesetzt, die mittels der Methode von Bradford bestimmt wurde (Bradford, M.M., Anal. Biochem. 72 (1976), 248-254). Die Ergebnisse der β-Galactosidase-Aktivitätsbestimmung zeigt Tabelle 2. Angegeben ist die pro Minute und mg total Protein freigesetzte Menge an o-Nitrophenol (gemessen als OD₄₂₀).

**Tabelle 2**

| β-Galactosidase-Expression | | |
|---|---|---|
| Plasmid | Messung Nr. | β-Galactosidaseaktivität (relative Einheiten, OD₄₂₀/mg min) |
| pAG-110 | 1 | 3,62 |
| | 2 | 3,54 |
| | 3 | 2,45 |
| pAG-111 | 1 | 3,07 |
| | 2 | 3,29 |
| | 3 | 3,63 |
| | 4 | 3,16 |
| pAG-112 | 1 | 1,89 |
| | 2 | 1,90 |
| | 3 | 1,79 |
| | 4 | 1,75 |
| pAG-113 | 1 | 0 |
| | 2 | 0 |
| pAG-114 | 1 | 0 |
| | 2 | 0 |
| pAG-115 | 1 | 0 |
| | 2 | 0 |

Sequenzprotokolle

Nr.1:

Art der Sequenz : Nucleotid
Sequenzlänge : 409 Basenpaare
Strangform : Einzelstrang
Topologie : linear
Art des Moleküls: Genom-DNA
Herkunft : A.gossypii
Eigenschaften : Promotorregion

AAGCTTGCCT CGTCCCNCGC GGGTCACCCG GCCAGCGACA TGGAGGCCCA GATACCCTCC 60

TTGACAGTCT TGACGTGCGC AGCTCACGGG GCATGATGTG ACTGTCGCCC GTACATTTAG 120

CCCATACATC CCCATGTATA ATCATTTGCA TCCATACATT TTGATGGCCG CGACGGCGCG 180

AAGCAAAAAT TACGGCTCCT CGCTGCAGAC CTGCGAGCAG GGAAACGCTC CCCTCAGCAG 240

ACGCGTTGAA TTCTCCCCAC GGCGCGCCCC TGTAGAGAAA TATAAAAGGT TAGGATTTGC 300

CACTGAGGTT CTTCTTTCAT ATACTTCCTT TTAAAATCTT GCTAGGATAC AGTTCTCACA 360

TCACATCCGA ACATAAACAA AAATGGGTAA GGAAAAGACT CACGTTAAC 409

Nr. 2
Sequenz des 2,1 kb EcoRI/BamHI Fragmentes mit dem offenen
Leseraster des TEF Gens

10 20 30 40 50 60
5' GAATTCTCCC CACGGCGCGC CCCTGTAGAG AAATATAAAA GGTTAGGATT TGCCACTGAG
70 80 90 100 110 120
GTTCTTCTTT CATATACTTC CTTTTAAAAT CTTGCTAGGA TACAGTTCTC ACATCACATC
130 140 150 160 170 180
CGAACATAAA CAAAAATGGG TAAGGAAAAG ACTCACGTTA ACGTTGTCGT CATCGGTCAC
190 200 210 220 230 240
GTCGACTCTG GTAAGTCTAC TACCACCGGT CACTTGATCT ACAAGTGTGG TGGTATTGAC
250 260 270 280 290 300
AAGAGAACCA TCGAGAAGTT CGAGAAGGAG GCTGCCGAGT TGGGTAAGGG TTCTTTCAAG
310 320 330 340 350 360
TACGCCTGGG TTTTGGACAA ATTGAAGGCT GAGAGAGAGA GAGGTATCAC CATCGACATT
370 380 390 400 410 420
GCGTTGTGGA AGTTCGAGAC TCCAAAGTAC CACGTCACTG TCATTGACGC CCCAGGCCAC
430 440 450 460 470 480
AGAGACTTCA TCAAGAACAT GATTACCGGT ACTTCTCAAG CTGACTGTGC CATCTTGATC
490 500 510 520 530 540
ATTGCTGGTG GTGTCGGTGA GTTCGAGGCT GGTATCTCCA AGGACGGTCA GACCAGAGAG
550 560 570 580 590 600
CACGCTTTGT TGGCTTACAC CTTGGGTGTC AAGCAGTTGA TCGTTGCCAT CAACAAGATG
610 620 630 640 650 660
GACTCCGTCA AGTGGGACGA GTCCAGATAC CAGGAGATTG TCAAGGAGAC CTCCAACTTC
670 680 690 700 710 720
ATCAAGAAGG TCGGTTACAA CCCTAAGACT GTTCCATTCG TTCCAATCTC CGGCTGGAAC
730 740 750 760 770 780
GGTGACAACA TGATTGAGGC CACCACCAAC GCCCCATGGT ACAAGGGCTG GGAGAAGGAG
790 800 810 820 830 840
ACCAAGGCTG GTGCCGTCAA GGGTAAGACC TTGTTGGAGG CCATTGACGC CATTGAGCCA
850 860 870 880 890 900
CCTGTCAGAC CAACTGACAA GGCATTGAGA TTGCCATTGC AGGATGTCTA CAAGATCGGT
910 920 930 940 950 960
GGTATTGGTA CGGTTCCAGT CGGCAGAGTC GAGACCGGTG TCATCAAGCC AGGTATGGTT
970 980 990 1000 1010 1020
GTTACCTTCG CCCCATCCGG TGTCACCACT GAAGTCAAGT CCGTCGAGAT GCACCACGAG
1030 1040 1050 1060 1070 1080
CAATTGGAGG AGGGTGTCCC AGGTGACAAC GTTGGTTTCA ACGTCAAGAA CGTCTCCGTC
1090 1100 1110 1120 1130 1140
AAGGAGATCA GAAGAGGTAA CGTTTGCGGT GACTCCAAGA ACGACCCACC AAAGGCTGCT
1150 1160 1170 1180 1190 1200
GAGTCCTTCA ACGCTACCGT CATTGTCTTG AACCACCCAG GTCAAATCTC TGCCGGTTAC
1210 1220 1230 1240 1250 1260
TCTCCAGTCT TGGACTGTCA CACTGCCCAC ATTGCTTGTA AGTTCGACGA GTTGTTGGAG
1270 1280 1290 1300 1310 1320
AAGAACGACA GAAGAACCGG TAAGAAGTTG GAAGACTCTC CAAAGTTCCT AAAGGCCGGT
1330 1340 1350 1360 1370 1380
GACGCTGCCA TGGTCAAGTT TGTCCCATCC AAGCCAATGT GTGTTGAGGC TTTCACCGAC
1390 1400 1410 1420 1430 1440
TACCCACCAT TGGGTAGATT CGCTGTCAGA GACATGAGAC AGACCGTTGC TGTCGGTGTC
1450 1460 1470 1480 1490 1500
ATCAAGTCTG TTGTCAAGTC CGACAAGGCT GGTAAGGTCA CCAAGGCCGC CCAAAAGGCT
1510 1520 1530 1540 1550 1560
GGTAAGAAAT AGAGTAACTG ACAATAAAAA GATTCTTGTT TTCAAGAACT TGTCATTTGT
1570 1580 1590 1600 1610 1620
ATAGTTTTTT TATATTGTAG TTGTTCTATT TTAATCAAAT GTTAGCGTGA TTTATATTTT

Nr. 2 (Fortsetzung)

1630 1640 1650 1660 1670 1680
TTTTGCCTCG ACATCATCTG CCCAGATGCG AAGTTAAGTG CGCAGAAAGT AATATCATGC
1690 1700 1710 1720 1730 1740
GTCAATCGTA TGTGAATGCT GGTCGCTATA CTGCTGTCGA TTCGATACTA ACGCCGCCAT
1750 1760 1770 1780 1790 1800
CCAGTGTCTA CCTGTCAAAT TTGCCAGCGT CAAATGCCTC CAGGATAGAA TATGCTCGAC
1810 1820 1830 1840 1850 1860
AACTGTTGAA GTCCATCAAC AAGGATAACC CATATGCTCT ATCGGCGGAG AAAACGTTGC
1870 1880 1890 1900 1910 1920
CAGAGCCGCT TCCTTCCGCA GACGTGCCCC TTCCACTGCT AGATGAGAAG TACGGGGTAG
1930 1940 1950 1960 1970 1980
TTAGTGTTTC CAGGCCTCGT AAATGCCGCA ATAAATGCTT CCTTGGGTTC GCTACGCCAT
1990 2000 2010 2020 2030 2040
CTCAGGCAGA CGAGTTTCTA CAAAACTTCA AGGACCGCCT TTTCATATAT GGCCACCAGG
2050 2060 2070 2080 2090
TCAATATAGA GCCAGCGAAC GATGATGCAT TCTGGTATAT TGAACGCGAG GATCC 3'

Nr. 3

Sequenz der Kanamycinresistenzgen - TEF Promotorregion - Fusion
(TEF Promotorregion Sequenzen unterstrichen)

10 20 30 40 50 60
5' GTCGACTGTA ATCCGGGCAG CGCAACGGAA CATTCATCAG TGTAAAAATG GAATCAATAA
70 80 90 100 110 120
AGCCCTGCGC AGCGCGCAGG GTCAGCCTGA ATACGCGTTT AATGACCAGC ACAGTCGTGA
130 140 150 160 170 180
TGGCAAGGTC AGAATAGCGC TGAGGTCTGC CTCGTGAAGA AGGTGTTGCT GACTCATACC
190 200 210 220 230 240
AGGCCTGAAT CGCCCCATCA TCCAGCCAGA AAGTGAGGGA GCCACGGTTG ATGAGAGCTT
250 260 270 280 290 300
TGTTGTAGGT GGACCAGTTG GTGATTTTGA ACTTTTGCTT TGCCACGGAA CGGTCTGCGT
310 320 330 340 350 360
TGTCGGGAAG ATGCGTGATC TGATCCTTCA ACTCAGCAAA AGTTCGATTT ATTCAACAAA
370 380 390 400 410 420
GCCACGTTGT GTCTCAAAAT CTCTGATGTT ACATTGCACA AGATAAAAAT ATATCATCAT
430 440 450 460 470 480
GAACAATAAA ACTGTCTGCT TACATAAACA GTAATACAAG GGGTGTTATG AGCCATATTC
490 500 510 520 530 540
AACGGGAAAC GTCTTGCTCG AAGCTTGCCT CGTCCCCGCC GGGTCACCCG GCCAGCGACA
550 560 570 580 590 600
TGGAGGCCCA GATACCCTCC TTGACAGTCT TGACGTGCGC AGCTCAGGGG GCATGATGTG
610 620 630 640 650 660
ACTGTCGCCC GTACATTTAG CCCATACATC CCCATGTATA ATCATTTGCA TCCATACATT
670 680 690 700 710 720
TTGATGGCCG CGACGGCGCG AAGCAAAAAT TACGGCTCCT CGCTGCAGAC CTGCGAGCAG
730 740 750 760 770 780
GGAAACGCTC CCCTCAGCAG ACGCGTTGAA TTCTCCCCAC GGCGCGCCCC TGTAGAGAAA
790 800 810 820 830 840
TATAAAAGGT TAGGATTTGC CACTGAGGTT CTTCTTTCAT ATACTTCCTT TTAAAATCTT
850 860 870 880 890 900
GCTAGGATAC AGTTCTCACA TCACATCCGA ACATAAACAA AAATGGGTAA GGAAAAGACT
910 920 930 940 950 960
CACGTTTCGA GGCCGCGATT AAATTCCAAC ATGGATGCTG ATTTATATGG GTATAAATGG
970 980 990 1000 1010 1020
GCTCGCGATA ATGTCGGGCA ATCAGGTGCG ACAATCTATC GATTGTATGG GAAGCCCGAT
1030 1040 1050 1060 1070 1080
GCGCCAGAGT TGTTTCTGAA ACATGGCAAA GGTAGCGTTG CCAATGATGT TACAGATGAG
1090 1100 1110 1120 1130 1140
ATGGTCAGAC TAAACTGGCT GACGGAATTT ATGCCTCTTC CGACCATCAA GCATTTTATC
1150 1160 1170 1180 1190 1200
CGTACTCCTG ATGATGCATG GTTACTCACC ACTGCGATCC CCGGGAAAAC AGCATTCCAG
1210 1220 1230 1240 1250 1260
GTATTAGAAG AATATCCTGA TTCAGGTGAA AATATTGTTG ATGCGCTGGC AGTGTTCCTG
1270 1280 1290 1300 1310 1320
CGCCGGTTGC ATTCGATTCC TGTTTGTAAT TGTCCTTTTA ACAGCGATCG CGTATTTCGT
1330 1340 1350 1360 1370 1380
CTCGCTCAGG CGCAATCACG AATGAATAAC GGTTTGGTTG ATGCGAGTGA TTTTGATGAC
1390 1400 1410 1420 1430 1440
GAGCGTAATG GCTGGCCTGT TGAACAAGTC TGGAAAGAAA TGCATAAGCT TTTGCCATTC
1450 1460 1470 1480 1490 1500
TCACCGGATT CAGTCGTCAC TCATGGTGAT TTCTCACTTG ATAACCTTAT TTTTGACGAG
1510 1520 1530 1540 1550 1560
GGGAAATTAA TAGGTTGTAT TGATGTTGGA CGAGTCGGAA TCGCAGACCG ATACCAGGAT
1570 1580 1590 1600 1610 1620
CTTGCCATCC TATGGAACTG CCTCGGTGAG TTTTCTCCTT CATTACAGAA ACGGCTTTTT

Nr. 3 (Fortsetzung)

1630 1640 1650 1660 1670 1680
CAAAAATATG GTATTGATAA TCCTGATATG AATAAATTGC AGTTTCATTT GATGCTCGAT
1690 1700 1710 1720 1730 1740
GAGTTTTTCT AATCAGAATT GGTTAATTGG TTGTAACACT GGCAGAGCAT TACGCTGACT
1750 1760 1770 1780 1790 1800
TGACGGGACG GCGGCTTTGT TGAATAAATC GAACTTTTGC TGAGTTGAAG GATCAGATCA
1810 1820 1830 1840 1850 1860
CGCATCTTCC CGACAACGCA GACCGTTCCG TGGCAAAGCA AAAGTTCAAA ATCACCAACT
1870 1880 1890 1900 1910 1920
GGTCCACCTA CAACAAAGCT CTCATCAACC GTGGCTCCCT CACTTTCTGG CTGGATGATG
1930 1940 1950 1960 1970 1980
GGGCGATTCA GGCCTGGTAT GAGTCAGCAA CACCTTCTTC ACGAGGCAGA CCTCAGCGCT
1990 2000 2010 2020 2030 2040
ATTCTGACCT TGCCATCACG ACTGTGCTGG TCATTAAACG CGTATTCAGG CTGACCCTGC
2050 2060 2070 2080 2090 2100
GCGCTGCGCA GGGCTTTATT GATTCCATTT TTACACTGAT GAATGTTCCG TTGCGCTGCC
2110
CGGATTACAG TCGAC 3'

Legenden zu den Figuren 1 bis 8
- Fig. 1:: Plasmid pAG-1. ARS: S.cerevisiae ARS1 Sequenz; 2 micron: EcoRI Fragment des S.cerevisiae 2µ Plasmids mit Replikationsursprung; URA3: S.cerevisiae URA3 Gen; G418r: G418 (Kanamycin)resistenz; geschlossener Pfeil: S.cerevisiae cycl-13 Promoter; Schwarze Box: S.cerevisiae CYC1 Terminator; offene Pfeile stellen die Transkriptionsrichtung dar.
- Fig. 2:: Plasmid pAG-2. amp: Ampicillinresistenz; 2 micron: EcoRI Fragment des S.cerevisiae 2µ Plasmids mit Replikationsursprung; URA3: S.cerevisiae URA3 Gen; G418r: G418 (Kanamycin)resistenz; ORI: Ursprung der Plasmidreplikation in E.coli; offene Pfeile stellen die Transkriptionsrichtung dar.
- Fig. 3:: Plasmid pAG-100. amp: Ampicillinresistenz; 2 micron: EcoRI Fragment des S.cerevisiae 2µ Plasmids mit Replikationsursprung; URA3: S.cerevisiae URA3 Gen; G418r: G418 (Kanamycin)resistenz; ORI: Ursprung der Plasmidreplikation in E.coli; geschlossener Pfeil: A.gossypii DNA Fragment mit TEF-Promotorregion; offene Pfeile stellen die Transkriptionsrichtung dar.
- Fig. 4:: Plasmid pAG-5. amp: Ampicillinresistenz;G418r: (Kanamycin)resistenz; ORI: Ursprung der Plasmidreplikation in E.coli; TEF: A.gossypii EcoRI/BamHI Fragment mit ORF für den Translationselongationsfaktor; offene Pfeile stellen die Transkriptionsrichtung dar.
- Fig. 5:: Plasmid pAG-101. amp: Ampicillinresistenz;G418r: G418 (Kanamycin)resistenz; ORI: Ursprung der Plasmidreplikation in E.coli; TEF: A.gossypii EcoRI/BamHI Fragment mit ORF für den Translationselongationsfaktor; geschlossener Pfeil: A.gossypii DNA Fragment mit TEF-Promotorregion; offene Pfeile stellen die Transkriptionsrichtung dar.
- Fig. 6:: Plasmid pPL1. amp: Ampicillinresistenzgen; M13+: Replikationsursprung für Einzelstrang-DNA-Isolierung; ori: Ursprung für Plasmidreplikation in E.coli ; lacZ: E. coli lacZ Gen; Prom: 1500 bp A.gossypii DNA Fragment mit der TEF-Promotorregion
- Fig. 7:: Plasmid pAG-110. 2µ: EcoRI Fragment des S.cerevisiae 2µ Plasmides mit Replikationsursprung; URA3: S.cerevisiae URA3 Gen; Prom: 1500 bp A.gossypii DNA Fragment mit der TEF-Promotorregion; lacZ: E.coli lacZ Gen; G418r: G418 (Kanamycin)resistenzgen; ori: Ursprung für Plasmidreplikation in E.coli ; amp: Ampicillinresistenzgen; geschlossener Pfeil: 1500 bp A.gossypii DNA Fragment mit der TEF-Promotorregion; offene Pfeile stellen die Transkriptionsrichtung dar.
- Fig. 8:: Plasmid pAG-111. 2µ: EcoRI Fragment des S.cerevisiae 2µ Plasmides mit Replikationsursprung; URA3: S.cerevisiae URA3 Gen; Prom: 403 bp A.gossypii DNA Fragment mit der TEF-Promotorregion; lacZ: E.coli lacZ Gen; G418r: G418 (Kanamycin)resistenzgen; ori: Ursprung für Plasmidreplikation in E.coli ; amp: Ampicillinresistenzgen; geschlossener Pfeil: A.gossypii DNA Fragment mit der TEF-Promotorregion; offene Pfeile stellen die Transkriptionsrichtung dar.
- Fig. 9:: Plasmid pPL2. amp: Ampicillinresistenzgen; M13+: Replikationsursprung für Einzelstrang-DNA-Isolierung; ori: Ursprung für Plasmidreplikation in E.coli ; lacZ: E. coli lacZ Gen; Prom: 294 bp A.gossypii DNA Fragment mit einem Teil der TEF-Promotorregion (270 bp).
- Fig. 10:: Plasmid pPL3. amp: Ampicillinresistenzgen; M13+: Replikationsursprung für Einzelstrang-DNA-Isolierung; ori: Ursprung für Plasmidreplikation in E.coli ; lacZ: E. coli lacZ Gen; Prom: 239 bp A.gossypii DNA Fragment mit einem Teil der TEF-Promotorregion (215 bp).
- Fig. 11:: Plasmid pPL4. amp: Ampicillinresistenzgen; M13+: Replikationsursprung für Einzelstrang-DNA-Isolierung; ori: Ursprung für Plasmidreplikation in E.coli ; lacZ: E. coli lacZ Gen; Prom: 158 bp A.gossypii DNA Fragment mit einem Teil der TEF-Promotorregion (134 bp).
- Fig. 12:: Plasmid pAG-112. 2µ: EcoRI Fragment des S.cerevisiae 2µ Plasmides mit Replikationsursprung; URA3: S.cerevisiae URA3 Gen; Prom: 294 bp A.gossypii DNA Fragment mit der TEF-Promotorregion; lacZ: E.coli lacZ Gen; G418r: G418 (Kanamycin)resistenzgen; ori: Ursprung für Plasmidreplikation in E.coli ; amp: Ampicillinresistenzgen; geschlossener Pfeil: A.gossypii DNA Fragment mit der TEF-Promotorregion; offene Pfeile stellen die Transkriptionsrichtung dar.
- Fig. 13:: Plasmid pAG-113. 2µ: EcoRI Fragment des S.cerevisiae 2µ Plasmides mit Replikationsursprung; URA3: S.cerevisiae URA3 Gen; Prom: 239 bp A.gossypii DNA Fragment mit der TEF-Promotorregion; lacZ: E.coli lacZ Gen; G418r: G418 (Kanamycin)resistenzgen; ori: Ursprung für Plasmidreplikation in E.coli ; amp: Ampicillinresistenzgen; geschlossener Pfeil: A.gossypii DNA Fragment mit der TEF-Promotorregion; offene Pfeile stellen die Transkriptionsrichtung dar.
- Fig. 14:: Plasmid pAG-114. 2µ: EcoRI Fragment des S.cerevisiae 2µ Plasmides mit Replikationsursprung; URA3: S.cerevisiae URA3 Gen; Prom: 158 bp A.gossypii DNA Fragment mit der TEF-Promotorregion; lacZ: E.coli lacZ Gen; G418r: G418 (Kanamycin)resistenzgen; ori: Ursprung für Plasmidreplikation in E.coli ; amp: Ampicillinresistenzgen; geschlossener Pfeil: A.gossypii DNA Fragment mit der TEF-Promotorregion; offene Pfeile stellen die Transkriptionsrichtung dar.
- Fig. 15:: Plasmid pAG-115. 2µ: EcoRI Fragment des S.cerevisiae 2µ Plasmides mit Replikationsursprung; URA3: S.cerevisiae URA3 Gen; lacZ: E.coli lacZ Gen; G418r: G418 (Kanamycin)resistenzgen; ori: Ursprung für Plasmidreplikation in E.coli ; amp: Ampicillinresistenzgen; offene Pfeile stellen die Transkriptionsrichtung dar.
- Fig. 16:: TEF-Promotorfragmente der β-Galactosidase-Expressionsplasmide.
- Fig. 17:: Nukleotidsequenz im ATG-Bereich und im Terminatorbereich von M13PT1, M13PT2, M13PT3, pAG-201, pAG-202, pAG-203.
- Fig. 18:: Plasmid pAG-201, pAG-202, pAG-203. 2 µ: ECORI Fragment des S. cerevisiae 2 µ Plasmides mit Replikationsursprung: Prom, Term: 751 bp A. gossypii DNA-Fragment mit der TEF-Promotor-Terminator-Fusion. G418r:G418 (Kanamycin)resistenzgen; ori: Ursprungspunkt der Plasmidreplikation in E. coli ; amp: Ampicillinresistenzgen; offene Pfeile stellen die Transkriptionsrichtung dar.
- Fig. 19:: Nukleotidsequenz von der Fusion aus Promotor und Terminator des TEF-Gens.

## Patentansprüche

1. Promotorregion des A. gossypii-Gens, welches den Translationselongatonsfaktor EF-1α kodiert, mit der im Sequenzprotokoll Nr. 1 angegebenen Nukleotidsequenz.

2. Pilze, die mit der Promotorregion gemäß Anspruch 1 genetisch verändert wurden.

3. Verwendung von Pilzen gemäß Anspruch 2 zur Herstellung von Proteinen.

4. Verwendung gemäß Anspruch 3, dadurch gekennzeichnet, daß der Pilz A. gossypii ist.

5. Verwendung von Pilzen gemäß Anspruch 2 zur konstitutiv verstärkten Expression von Genen der Vitamin B₂-Biosynthese oder Genen, die für die Überproduktion von Vitamin B₂ verantwortlich sind.

6. Terminatorregion des A. gossypii-Gens, welches den Translationselongationsfaktor EF-1α kodiert, mit der im Sequenzprotokoll Nr. 2, Position 1513-2095 angegebenen Nukleotidsequenz, die 3'-terminal verkürzt sein kann, unter der Voraussetzung, daß diese verkürzte Terminatorregion noch funktionell effizient ist.

## Claims

1. Promoter region of the A. gossypii gene which encodes the translation elongation factor EF-1α, having the nucleotide sequence indicated in sequence listing No.1.

2. Fungi which have been genetically modified with the promoter region as claimed in claim 1.

3. The use of fungi as claimed in claim 2 for producing proteins.

4. The use as claimed in claim 3, wherein the fungus is A. gossypii.

5. The use of fungi as claimed in claim 2 for the constitutively enhanced expression of genes of vitamin B₂ biosynthesis or of genes which are responsible for overproduction of vitamin B₂.

6. Terminator region of the A. gossypii gene which encodes the translation elongation factor EF-1α, having the nucleotide sequence indicated in sequence listing No.2, position 1513-2095, which may have a 3'- terminal truncation provided that this truncated terminator region is still functionally efficient.

## Revendications

1. Région promoteur du gène A. gossypii qui code le facteur translation-élongation EF-1α, avec la séquence de nucléotides indiquée dans le protocole des séquences No.1.

2. Champignons qui ont été modifiés génétiquement avec la région promoteur selon la revendication 1.

3. Utilisation de champignons selon la revendication 2 pour la préparation de protéines.

4. Utilisation selon la revendication 3, caractérisée par le fait que le champignon est A. gossypii.

5. Utilisation de champignons selon la revendication 2 pour l'expression constitutivement renforcée de gènes de la biosynthèse de la vitamine B₂ ou de gènes responsables de la surproduction de vitamine B₂.

6. Région terminale du gène A. gossypii codant le facteur translationélongation EF-1α, avec la séquence de nucléotides indiquée dans le protocole No 2, position 1513-2095, qui peut être raccourcie à l'extrémité 3'-terminale, étant entendu que cette région terminale raccourcie est encore fonctionnellement efficace.
